Europäisches Patentamt

European Patent Office

Office européen des brevets

(11) Publication number: **0 311 438 B1**

# EUROPEAN PATENT SPECIFICATION

(45) Date of publication of patent specification: **28.12.94**

(51) Int. Cl.5: **C12P 21/00**, C12N 15/00, C12N 5/00, A61K 39/395, A61K 37/02, C07K 15/04, //(C12P21/00,C12R1:91)

(21) Application number: **88309398.1**

(22) Date of filing: **07.10.88**

(54) Antigen capable of binding to cancer cells.

(30) Priority: **08.10.87 US 115739**

(43) Date of publication of application:
**12.04.89 Bulletin 89/15**

(45) Publication of the grant of the patent:
**28.12.94 Bulletin 94/52**

(84) Designated Contracting States:
**AT BE CH DE ES FR GB GR IT LI LU NL SE**

(56) References cited:

**British Medical Bulletin, vol. 40, no. 3, 1984, pp. 240-246; R.O. Dillman et al.**

**Hybridoma, vol. 1, no. 3, 1982, pp. 301-311, Mary Ann Liebert Inc. Publishers; R. Billing et al.**

**Transplantation Proceedings, vol. 15, no. 1, March 1983, pp. 649-650, Grune & Stratton Inc.; R. Billing et al.**

**Proc. Int. Conf. Monoclonal Antibodies, 1983,**

**pp. 85-90; R. Billing et al.**

(73) Proprietor: **Billing, Ronald James**
**2930-104 Norman Strasse Road**
**San Marcos**
**California 92069 (US)**

(72) Inventor: **Billing, Ronald James**
**2930-104 Norman Strasse Road**
**San Marcos**
**California 92069 (US)**

(74) Representative: **Newstead, Michael John et al**
**Page Hargrave**
**Temple Gate House**
**Temple Gate**
**Bristol BS1 6PL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an antigen capable of binding to tumor cells.

Monoclonal antibodies (MAs) are secreted from hybrid cells called hybridomas. The hybrid cells are made by fusing spleen cells from immunised normal mice and cancer cells called myelomas from the same species. The mice are immunised with cells that express the antigen that one desires a monoclonal antibody (MA) against. Following the fusion hybridomas are selected for those that secrete antibodies against desirable antigens. The hybridoma inherits the normal cell's ability to manufacture antibodies and the cancer cell's capacity to divide. This produces a cell line grown in culture that continuously secretes an unlimited supply of homogeneous antibodies. Therefore mass production of the antibody is a feature suitable for therapeutic uses. The antigenic determinant against which the MA reacts determines the potential value of the hybridoma and the MA.

Hybridoma Vol. 1, No. 3, 1982, pp 303-311 and British Medical Bulletin, 1984, Vol. 40, No. 3, pp 240-246 describe an MA produced from a mouse hybridoma (CBL1) and a particular transferrin receptor thereof. The MA is known as CBL1 monoclonal antibody and, while unreactive to the majority of normal tissue and blood cells, reacts with leukaemia cell lines whereas certain other MAs such as Tac (Uchiyama et al., J. Immunol. 1981, 126 pp 1393-1397) do not.

The present invention is based on the finding that the CBL1 MA reacts with a unique antigen which is found almost entirely on actively dividing cells such as stimulated lymphocytes (called blast cells) and cancer cells of all types. The antigen is located on the cell membrane and controls the growth and division of these cells.

According to the present invention, therefore, there is provided a partially purified antigen of vertebrate source that has the following properties:

(a) it functions as an autocrine growth factor produced by tumor cells and activated lymphocytes,

(b) it binds to the surface membrane of tumor cells and stimulates the growth of these cells and cells of the lymphoid series,

(c) it is present on the cell membrane and within the cytoplasm of tumor cells and activated lymphocytes,

(d) it is present in the cytoplasm of unstimulated normal peripheral blood lymphocytes but when these cells are stimulated by antigens or by mitogens, said antigen appears also on cell membrane,

(e) it is present on lymphocytes activated in vitro by mitogens,

(f) its molecular weight is approximately 15,000 daltons,

(g) it is capable of binding to CBL1 monoclonal antibody which is produced by the hybridoma cell line having ATCC number HB 8214.

Growth factor is a general term for molecules that stimulate and control the growth of cells mainly by binding initially to the cell membrane. Most cells in the body are in a slow growth or non dividing state. The majority of peripheral blood lymphocytes are not dividing unless they are activated by foreign antigens. Cancer cells are rapidly dividing cells of uncontrolled growth. Growth factor binds to the cell membrane and allows nutrients to enter the cell which promote these changes in growth characteristics (Cancer, Devita et al., 1985, p 50 supra). Cells growing in cultured nutrient medium require serum as a source of growth factors, (Gospodarowit D. et al., Ann. Rev. Biochem. 451, 531 1976). Several of these serum growth factors have now been identified and purified; some are hormones such as insulin, erythropoietin and others are polypeptides that promote growth of certain normal tissue cells such as fibroblast growth factor, epidermal growth factor, nerve growth factor, platelet derived growth factor, transferrin. Interleukin 2 is a growth factor for activated normal T lymphocytes. Autocrine growth factors are produced by cancer cells themselves possibly from viral DNA integrated into the cell genetic material. They are shed into the cellular environment such as culture medium and cause further growth and cell division (Cancer, Edited by V.T. Devita et al., 1985 p 50 supra).

The antigen of the present invention is a 15k dalton molecule which is not resistant to proteolysis and is located both on the cell membrane and in the cytoplasm of positive cells. It has the properties of an autocrine growth factor. It is secreted into the culture media by cancer cells and stimulates the growth of cancer cells in general.

By binding to the antigen on the surface of a tumor cell in culture, the CBL1 MA will prevent the growth of the tumor cell. The mechanism for this inhibition of growth appears to be that the MA neutralises the antigenic determinant and thus inhibits its role of stimulating cell growth when bound to a receptor molecule on the cell surface. The antigen can be purified from culture media and shown to increase the growth rate of cultured tumor cells. This increase in growth rate can be inhibited by addition of the CBL1 MA to the culture media.

2

The antigen [termed hereafter CAGF (cancer associated growth factor)] is found in the cytoplasm of normal lymphocytes in the blood but not on the surface membrane of these cells. Following stimulation of normal lymphocytes with the mitogen, phytohemaglutinin (PHA), the CAGF antigen migrates within a few hours to the cell membrane. PHA stimulated cells are called activated or blast cells and unlike the majority of lymphocytes they proceed to make DNA and divide after 3 or 4 days. This indicates that CAGF is functioning at the cell membrane to control cell growth in some yet unknown way. CBL1 will bind to the membrane of activated lymphocytes but not to the membrane of normal unstimulated lymphocytes in the blood of healthy donors.

When lymphocytes from two unrelated donors are mixed in tissue culture medium a reaction occurs called the mixed lymphocyte reaction (MLR). In MLR, unstimulated lymphocytes begin to become blast cells and express CAGF on their surface membrane. As in the use of PHA activated cells, CBL1 will bind to the cell membranes of MLR activated cells but not to the membranes of normal lymphocytes.

A biological specimen may be assayed for the presence of the antigen, or its cell receptor in tissues, on cells or in body fluids, by an assay method comprising the steps of incubating the specimen with labelled or unlabelled CBL1 antibody, and determining whether or not an immune complex is formed.

According to a further aspect, the invention provides the new antigen for use as a diagnostic or therapeutic agent. The following discussion serves to illustrate these uses, both by reference to the use of the antigen as such (as a growth factor to stimulate cell growth) or as an intermediary for facilitating coupling of a molecule or substance to a cell. In this aspect the invention has application against such diseases as e.g. (a) solid tumors and acute leukemias, (b) autoimmune diseases, (c) transplant rejections, and (d) allergic diseases.

**Binding of CBL1 MA to cells**

These tests done outside the body, i.e. in vitro, relate to the observed therapeutic uses of the CBL1 MA. The MLR test is an in vitro model of what is happening in a human transplant situation. The recipient of the transplanted organ reacts to the foreign graft by producing activated lymphocytes that attack the graft causing rejection of the transplanted organ. CBL1 will dramatically reverse transplant rejection without side effects to the patient. The most probable explanation is that the MA specifically destroys the activated lymphocytes that are mainly responsible for the rejection process but does not kill normal cells. This includes normal white blood cells which are needed to protect the patient against infections.

PHA stimulation of lymphocytes in vitro is a model of what happens in the body during allergic reactions and autoimmune diseases. In these diseases activated lymphocytes are produced in response to a foreign allergen. In the case of autoimmune diseases the body reacts to a molecule that is a functional part of the body itself. For example in rheumatoid arthritis the patient makes antibodies to his own immunoglobulin molecules, in multiple sclerosis there is an autoimmune attack against parts of the nervous system and in systemic lupus antibodies are made against DNA. Activated lymphocytes are thought to be involved as an important mediator of the immune disease process. Cell membrane binding tests have shown that patients with these diseases have a significant increase in CBL1 positive cells. Therefore by killing these activated cells the CBL1 may have therapeutic applications in patients with these disorders.

Unlike MAs described in prior art CBL1 has no side effects on patients or on nonhuman primates (Rhesus monkeys) treated. Therefore CBL1 can be used as a safe therapeutic agent to treat transplant rejection, autoimmune diseases or cancer. The reason for the lack of side effects appears to be that CBL1 will only kill those cells that express the CBL1 antigen (CAGF) on the cell surface membrane and these cells are restricted essentially to activated lymphocytes and tumor cells. The normal cells in blood and body tissues are essentially negative. Therefore the antibody will attack mainly those cells that are causing the disease leaving normal cells unaffected.

The CBL1 MA kills cells by binding to the specific antigen CAGF present on the cell surface. Serum complement factors react with the MA bound to antigen on the cell surface to lyse the cell. This is termed complement dependent cell cytotoxicity. B lymphocytes can also be used to kill cells to which MA is bound. Cells with antigen in the cytoplasm but not on the cell surface would not be killed.

The MA CBL1 has been used both intravenously (iv) and intramuscularly (im) to treat over 30 patients and no side effects were observed. The CBL1 MA was first used to prolong skin transplants in Rhesus monkeys. There was a significant increase in graft survival without any observable reactions against normal body cells. Because of the safety of the MA in the animal studies, CBL1 has been used in several clinical trials. It reversed acute kidney transplant rejection in 17 of 19 patients who had failed conventional drug therapy. In another study corneal graft rejections were reversed in six patients who had very poor or no vision. Unlike reagents described in prior art there were no significant side effects observed and the

EP 0 311 438 B1

reversal of graft rejection appeared to be permanent.

Preparation of Antigen

Hybridomas and monoclonal antibodies can be obtained substantially following the well-known procedure of Kohler and Milstein, Eur. J. Immunol. (1976) 6:511-591. The method involves hyperimmunisation of a mammalian host,e.g., a mouse, with a tumor cell line (CEM). Shortly after the last immunisation, the host spleen is removed and the spleen cells fused with myeloma cells to produce hybridomas having a range of specificities. The hybridomas are selected on HAT media, and the supernatants screened for antibodies having the desired specificity. Those colonies which produce the desired antibodies may then be cloned by limiting dilution and grown out in flasks. Production of the desired antibodies may be enhanced by intraperitoneal injection of the hybridoma into a mammalian host to produce high-titered ascites fluid. In contrast to antisera derived directly from an immunised host, the monoclonal antibodies are substantially free from immunoglobulins having differing specificities.

The antibodies can be used to isolate the antigenic determinant by the method of affinity chromatography, (see booklet,"Affinity Chromatography" by Pharmacia Fine Chemicals, Uppsala, Sweden). Essentially the antibody is bound covalently to Sepharose$^R$ beads to prepare an immunosorbent column. By lysing the CEM cells, separating the cellular debris, and then passing the supernatant through the column, the molecules displaying the determinant will bind to the column. After washing the column to remove nonspecifically bound proteins, the antigen or determinant may be eluted, using detergents, urea, etc. in programmed gradient elution. The antigen may then be isolated and used to hyperimmunise an appropriate host followed by fusion of splenocytes with a fusion partner and screening for the desired hybridoma(s). In this way, monoclonal antibodies having substantially identical specificities, but which differ in class, such as IgM, IgG, IgA, IgE and IgD, may be obtained. In addition, antibodies from a variety of different hosts can be obtained.

Diagnostic and Therapeutic Uses of the Antigen

The purified antigen can be used for diagnostic and therapeutic procedures and as a growth factor to stimulate cell growth.

(a) Binding CBL1 antibodies to tumor cells

The subject antibodies can be used directly as therapeutic agents. For such use, it is desirable to employ antibodies of class IgM, IgG2a, IgG2b, or IgG3 which activate the host's own complement system to reduce the population of activated lymphocytes or cancer cells. Alternatively, antibodies of any class may be coupled to cytotoxic agents to provide a composition lethal to target cells but nonlethal to the remainder of the normal peripheral blood poopulatlon. Suitable cytotoxic agents include chlorambucil (Ghose et al. (1975), Cancer 36:1646-1657), whole diptheria toxin (Moolten et al.(1972) J. Natl. Cancer Inst. 49:1059-1062; Moolten et al. ibid 55:473-477; and Thorpe et al. (1978) Nature 271:752-755), and the A chain of ricin toxin (Oeltmann et al. (1979) J. Biol. Chem. 254:1022-1027; and Oeltmann et al., ibid, 254:1028-1032). For administration to a patient, the antibodies are dissolved in physiologically acceptable media, such as saline, or phosphate buffered saline and administered in a variety of ways, conveniently intravenously or intramuscularly.

The subject antibodies will also find use in detection of malignant cells and in immunological classification of leukemia. Leukemias can be divided into immunological subsets depending on the particular surface antigens displayed by the malignant cells. For example, CBL1 antibody is reactive with three out of the four generally recognised morphological types of leukemia, being nonreactive with chronic lymphocytic leukemia (CLL). CBL1 would thus be useful, in combination with other antibodies having different specificities, in clinical diagnosis of a patient having leukemia. Malignant lymphocyte cells which are incapable of reacting with CBL1 would be presumed to be CLL.

(b) Binding cancer detection systems to tumor cells

A further use of the invention is to detect cancer cells in body tissues by binding of the antibody or antigen coupled to a detection system. A detection system could be a radioactive molecule such as $I^{125}$ covalently bound to the antibody or antigen. The latter complex would specifically bind to the cancer cell following infusion of the radioactively labelled antibody or antigen into the blood. Complex bound to cancer

4

cells could then be detected by X-rays or radio-imaging instruments or gamma ray counting. Another detection system would be an enzyme such as peroxidase that could detect antibody bound to cancer cells in tissue sections by changes in the color of a substrate dye. This would be useful in detecting cancer cells in biopsy material.

(c) Binding toxins to tumor cells via antigen

The CBL1 antigenic determinant has medical uses because as an autocrine growth factor it binds to tumor cell membranes. Therefore it can be linked to toxins such as ricin, (and others described above), and used as an anti-tissue rejection or cancer reagent in the same manner as an antibody.

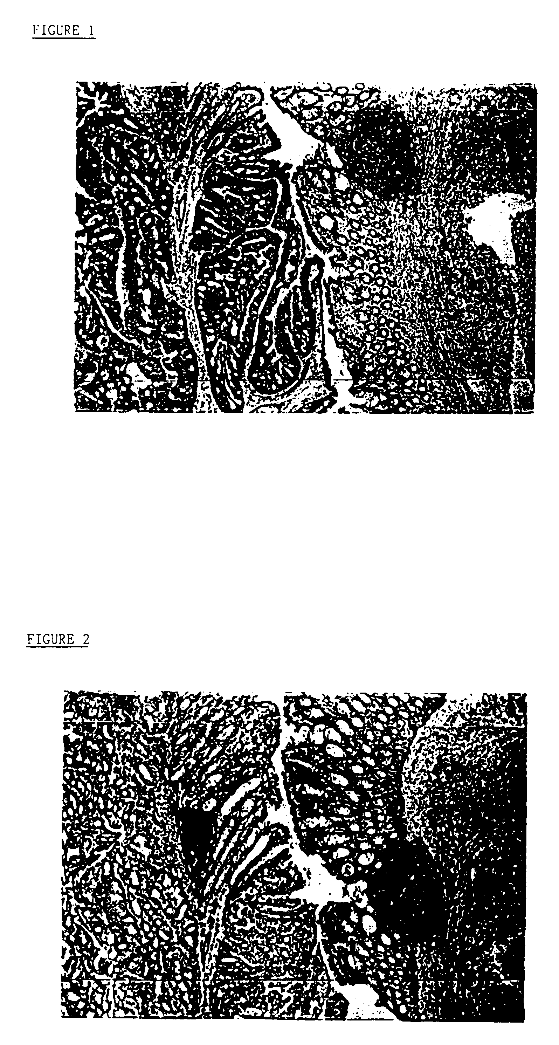

The CBL1 antibody binds a different epitope to the one that binds the CAGF antigen, i.e. growth factor, to the cell membrane. The CBL1 antibody binds to the epitope of the growth factor that protrudes from the cell membrane and therefore the antibody can bind to the cell surface by means of the antigenic epitope of the growth factor that protrudes from the cell membrane. Another part or epitope of the growth factor binds to the cell membrane. Cells that are not dividing will not have the membrane receptors to bind the CBL1 growth factor and therefore will not bind the CBL1 antigen or antibody. (Fig.1).

A further use for the invention is the treatment of cancer by infusion of the antibody. It can bind to and kill tumor cells directly by complement dependent cytotoxicity or by binding to the antigen and killing the tumor cell indirectly by depriving it of an essential growth factor.

Autoimmune diseases and allergic reactions are those in which the patient's own immune system produces an immune response against his own tissues or foreign allergens. These reactions can be mediated by activated lymphocytes and therefore may be treatable with antibodies specific for these cells.

The subject hybridoma was deposited on February 24th 1983 at the ATCC, American Type Culture Collection, 12401 Parklawn Dr., Rockville, Md 20852 and was given the ATCC number HB 8214.

DETAILED DESCRIPTION OF THE INVENTION

Method of production of the hybridoma and monoclonal antibody.

Two 6 week old female Balb/c mice (Simonsen Labs) were immunised intravenously with $2 \times 10^6$ acute leukemia cells weekly for 3 weeks. Three days after the final injection the spleens were removed sterilely into RPMI medium and a single cell suspension of splenocytes made by teasing the tissue in RPMI medium with a scalpel. $2 \times 10^8$ spleen cells were fused with $50 \times 10^6$ P3 x 63 Ag8 651 myeloma cells (from ATCC) in 50% polyethylene glycol 4000 in RPMI medium. The fused cells were plated in six microtest plates containing 96 wells and allowed to grow in RPMI medium with 20% fetal calf serum containing hypoxanthine, aminopterine and thymidine. After three weeks, hybridoma colonies had grown out and supernatants from the microtest plate wells were screened by microcytotoxicity against the immunising cell and normal lymphocytes. One microtest plate from 600 produced a supernatant from a hybridoma that showed reactivity against tumor cells but not normal cells. This hybridoma was cloned by the limiting dilution method, expanded and frozen in liquid nitrogen. Other cloned cells were grown to produce ascites fluid in the peritoneal cavity of pristane primed Balb/c mice. $20 \times 10^6$ cells per mouse produced 4-6 ml ascitic fluid containing high levels of monoclonal antibody. The fusion and purification techniques are described in detail in Monoclonal Antibodies, 1980 ED by R. H. Kennet et al. PLenum Press, New York. The antibody was purified by ammonium sulfate (40%) precipitation followed by exclusion gel filtration on S300 (Sephadex$^R$).Materials used in characterisation of CBL1.

Leukemia cells: Heparinised peripheral blood samples were drawn from children and adults with active leukemia. The acute lymphocytic leukemia (ALL) and acute myelocytic leukemia (AML) patients had peripheral blood blast counts greater than 90%. Leukemia cells from patients and peripheral blood lymphocytes from healthy donors were isolated by Ficoll-Hypaque$^R$ density gradient centrifugation. Leukemia cells were stored in liquid nitrogen.

Cell lines were grown in suspension cultures in RPMI 1640 containing 10% heat-inactivated fetal calf serum. Most were obtained from ATCC, Rockville, MD.

T and B lymphocytes: T and B lymphocytes were prepared from whole lymphocytes by the nylon-wool method. Danilovs et al. (1980) Histocompatibility Testing 1980, Terasaki (ed.) UCLA Tissue Typing Laboratory, Los Angeles, CA., pp. 287-288.

PHA Blasts: Whole peripheral blood lymphocytes isolated by the Ficoll-Hypaque$^R$ technique were cultured at $2.5 \times 10^6$ in media 199 (M199) with 20% human AB serum (heat inactivated) at 37°C with 50ug/ml of Difco PHA under sterile conditions for 3-6 days. Control lymphocytes were incubated in M199 with 20% human AB serum without PHA. At the end of the culture period, cells were removed , washed and tested by microcytotoxicity against the blast sera.

Monocytes: Monocytes were isolated using a Percoll[R] density gradient. Gutierrez et al. (1979) Immunol. Meth. 29: 57-63. Briefly, the thrombin pellet from lymphocyte isolation containing monocytes, platelets, and granulocytes was washed and resuspended in 65% Percoll[R] in PBS (by volume). Then 55% Percoll[R], 40% Percoll[R] and McCoy's media were layered, respectively , over the suspension and centrifuged at 3200 x g for 10 min with the brake off. The monocytes were recovered from the 40-55% Percoll[R] interface, diluted with media and washed.

Granulocytes: Granulocytes were isolated from Ficoll[R] pellets by removing RBCs by agglutination. The Ficoll[R] pellet from lymphocyte isolation containing granulocytes and RBCs was suspended in McCoy's media and centrifuged at 3000 x g for 1 min and the buffy coat from the pellet removed. This process was repeated until the buffy coat was sufficiently enriched for granulocytes. The appropriate agglutinin (anti-A, -B, -AB, -O, and -H) was added and allowed to agglutinate fully. The clumps were then spun at 1500 x g for less than 1 sec and the supernatant layered over Ficoll[R] (1.3545g/ml) and centrifuged at 3000 x g for 2 min. The granulocyte-enriched pellet was then washed and tested against antiblast sera. (This entire process was done in Fisher tubes using a Fisher, Model 59, centrifuge.)

Methods used to characterise CBL1 reactivity.

A. Cytotoxicity. Cytotoxicity tests measure the extent of killing of a cell population by cytotoxic monoclonal antibodies. The antibody binds to the cell surface and the cell is lysed or killed by complement components in serum. A dye and a microscope are used to count dead cells. Cytotoxicity is a natural method used by the body to kill tumor cells reactive with infused monoclonal antibodies. A rapid cytotoxic screening test was used to determine the specificity of the subject antibody for tumor cells and not normal cells. One microliter of antibody at dilutions from 1:10 to $1:10^7$ were added to 2000 viable cells in wells of a microtiter plate. Following incubation of 30 mins that allows the antibody time to bind to the cell, rabbit serum (5ul) was added for one hour. The complement components in the rabbit serum killed cells that bound antibody but not cells that did not bind antibody. The dead cells are identified by adding a red colored dye (eosin) which enters dead cells but not living cells. The number of dead cells can be counted using a light microscope at 200x magnification. (Billing et al 1979 Immunol. Immunopathol. 13, 435).

B. Tissue Staining. A different method of antibody binding test was used to identify the positive cells in tissue sections from patients. A thin slice (6-8 microns) of the frozen organ tissues was cut by a cryostat and placed on a microscope slide. The tissue sections were fixed in acetone for 10 mins. Dilutions of the monoclonal antibody from 1:10 to 1:10,000 were added to the slides. They were incubated for 15 mins with a second antibody, peroxidase linked goat anti-mouse immunoglobulin. Following a wash step the substrate for the peroxidase enzyme, aminoethyl carbazole (AEC), was added for 10 mins. After a final wash the tissues were counterstained with Mayer's hematoxylin and preserved in aquamount (Lerner Laboratories). The cells that contained the antigen stained a reddish brown color whereas negative cells and tissue stained pale blue due to the counterstain, Mayer hematoxylin. Control studies included staining with normal mouse ascites and a mouse monoclonal antibody against normal human leukocytes.

C. Molecular weight of the antigenic determinant. The method of Immuno-precipitation and Polyacrylamide Gel Electrophoresis was used. Cells ($5 \times 10^6$ per experiment) were labelled with $^{125}I$ by the iodogen technique of Markwell and Fox (1978) Biochemistry 17: 4807-4817, solubilised with 300ml 0.5% Nonidet[R] P40, and immunoprecipitated with 10ul CBL1 bound to 40ul rabbit anti-mouse IgM-protein A Sepharose 4B. After washing three times with PBS, the precipitated antigens were released from the protein A by adding 50ul 2% SDS. They were boiled for 2 mins with or without dithiothreitol and run on 12% gels. Fifty 2mm slices from each gel were counted on a gamma counter. Standard proteins of known molecular weight were also run in order to calculate the molecular weight of the precipitated antigens. The gel system was that originally described by King and Laemmli (1971) J. Mol. Biol. 62: 467-480, and used by Billing et al. (1978) J. Natl. Cancer Inst. 61: 423-429. The molecular weight and protein standards used were lysozyme 14,400, soybean trypsin inhibitor 21,500, carbonic anhydrase 31,000, ovalbumin 45,000, bovine serum albumin 68,000, phosphorylase B 92,900, and B-galactosidase 116,500.

The molecular weight of the antigen detected by the subject antibody was estimated from a graph of the migration distances of the standards plotted against their molecular weight. Tumor cells or spent culture media were used as a source of the antigen.

SDS electrophoresis of the monoclonal antibody and immunodiffusion studies with antimouse immunoglobulin subtype antisera demonstrated that the monoclonal antibody was of the IgM subclass.

The antigen can be purified from conditioned media and NP40 cell lysates (described above) by affinity chromatography using purified antibody bound to Sepharose or latex beads (Affinity chromatography by Pharmacia). Following washing of the column with phosphate buffered saline the antigen can be eluted by chaotropic agents or glycine buffer pH 2.8.

BRIEF DESCRIPTION OF THE DRAWINGS

The following Figures and Tables will provide experimental results from which the characteristics of the MA and its antigen were determined. They will also give additional evidence of the uses of the invention described in the specification.

Table 1 shows the types of cells that react with the MA by cytotoxicity testing.

Table 2 gives the percentages of lymphocytes that express the CAGF antigenic determinant before and after stimulation with a mitogen PHA.

Table 3 shows growth study results with CBL1.

Table 4 shows allogeneic skin graft survival times in Rhesus monkeys treated with the MA, CBL1.

Fig 1-4 shows photographs of staining of tissue sections with CBL1.

Fig 5-7 shows rejection course monitored by serum creatinine of kidney transplants.

Fig 8 shows the monitoring of blood lymphocyte counts during MA treatment.

Fig 9 shows a stained skin section containing areas of necrotic tissue. The patient (EC) was treated with MA.

The description of the invention will be expanded below.

A. Characterisation of the CBL1 reactivity.

Table 1 shows the results of testing the MA for killing of human cell types. The cells that were highly positive were cultured solid tumor cells, leukemia cells, activated lymphocytes and monocytes. Most normal non-dividing tissue cells were not reactive. Therefore the antibody is reactive with an antigen that is present essentially on cells that are dividing. Activated lymphocytes are dividing under normal immunological conditions in that they have been stimulated to divide as part of the normal immune response to a foreign antigen. Cancer cells are dividing uncontrollably.

The photographs (Fig 1-4) show tissue sections of colon and breast cancer tissue stained with CBL1 and goat anti-mouse immunoglobulin bound to peroxidase. The substrate is aminoethyl carbazole (AEC) which gives a positive reddish-brown stain. The tissues are counter-stained with Mayer hematoxylin which shows a background intensity of blue proportional to the cell density. The photographs demonstrate the specificity of CBL1 for tumor tissue.

Fig 1 shows colon carcinoma stained with CBL1 Magnification is 250x. The malignant tissue that is present on the left side of the photograph is stained reddish-brown showing reaction with CBL1, whereas the normal colon endothelium on the right is negative. The large blue mass at the top right of center is a lymph node showing no reaction with CBL1

Fig 2 shows the same colon carcinoma section stained with mouse monoclonal antibody against normal or human lymphocyte antigen (T29). Magnification 250x. This is a negative control for the CBL1 T29 does not stain the tumor tissue on the left but stains lymphoid cell infiltrates in the endothelium on the right and the normal lymph node (lower right).

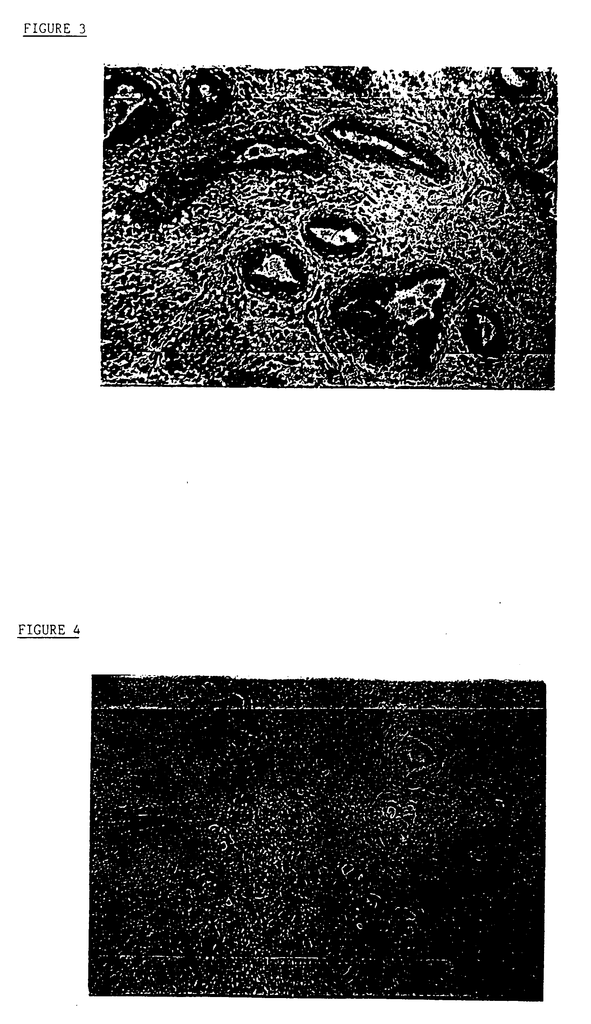

Fig 3 shows breast cancer tissue stained with CBL1 Magnification 100x. The malignant cells surrounding the glandular ducts are CBL1 positive whereas the normal stroma is CBL1 negative.

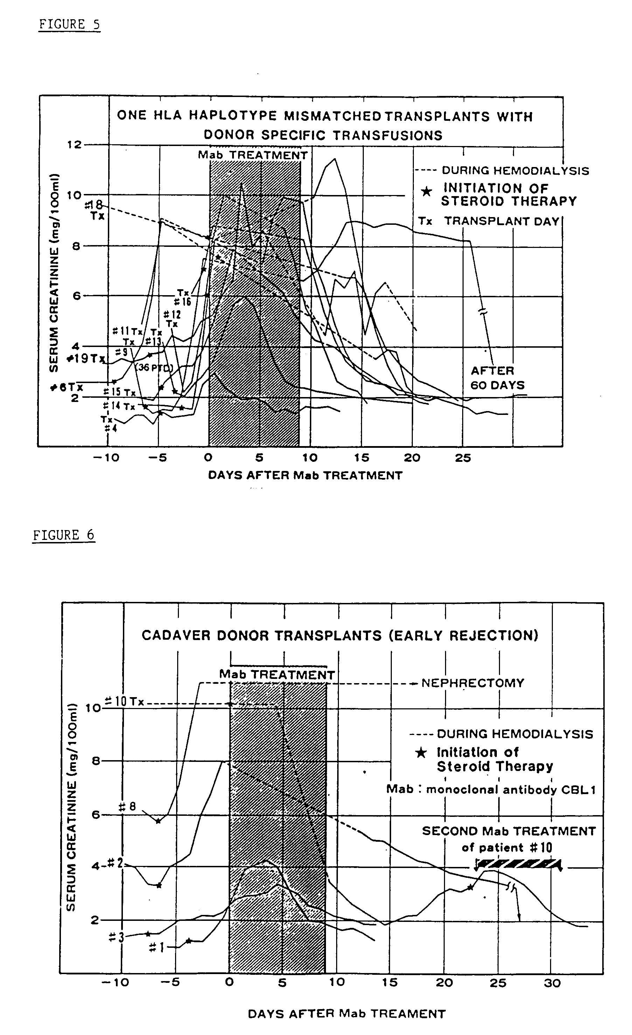

Fig 4 shows normal breast tissue stained with CBL1 Magnification 100X. There are no CBL1 stained cells.

In addition to the tissues shown in the photographs, the following different carcinomas were stained positive for CBL1. The number in parenthesis represents the number of patients tested:- colon (6), rectal (1), esophageal (2), breast (5), kidney (1), meningioma (3), pancreatic (1), adrenal (1). Normal tissue from healthy donors and normal tissue adajcent to malignant tissue was not stained and therefore unreactive with CBL1

B. Presence of antigenic determinant on activated lymphocytes.

Table 2 illustrates by immunoperoxidase staining of lymphocytes with the CBL1 antibody that the CBL1 antigen is present in the cytoplasm of unstimulated normal lymphocytes. It is not present on the cell surface of these cells. Five hours following stimulation of the lymphocytes with PHA or allogeneic lymphocytes, the CBL1 antigen is present on the cell surface membrane in addition to also being in the cytoplasm. Therefore the antigen must have some function at the cell membrane of activated or dividing cells. This function as described below appears to be that of a growth promoting agent or growth factor. As a control, the OKT9 antibody that reacts with blast cells was present only on the surface of activated cells. The CBL1 antigen is unique in that it is found in the cytoplasm but not the cell surface of non-dividing lymphocytes and it is present on the cell surface of dividing cells such as activated lymphocytes and cancer cells.

C. Growth factor studies.

The CBL1 antibody inhibits the division of tumor cells growing in tissue culture by binding to a soluble antigen in the culture medium (Table 3).

Serum free conditioned media supernatant from growing tumor cells at a density of $1 \times 10^6$ / ml will support the growth of live tumor cells growing at a low density ($10^5$ / ml), Without 20% conditioned media supernatant these tumor cell lines will die. They appear to need a growth factor which is present in the conditioned media. This growth factor could be blocked by the addition of 10-50 micrograms / ml of pure CBL1 antibody but not by three other mouse monoclonal antibodies against lymphoid cell lines. The conditioned media growth factor could be removed and isolated from the conditioned media with CBL1 affinity coated protein A beads as described on pages 12, 13. The conditioned media growth factor and the cell surface antigen detected by the CBL1 monoclonal antibody have the same molecular weight of approximately 15,000 daltons. This cancer cell related growth factor appears to be an autocrine factor meaning that it is produced and secreted by the tumor cells themselves into the cell media and stimulates growth of other cancer cells. It can be purified from growth media by ammonium sulfate precipitation followed by gel chromatography on G50 Sephadex$^R$. Treatment of the antigen with proteolytic enzymes will destroy its activity. A similar growth factor is present in mouse ascitic fluid and it may be associated with vertebrate cancers in general.

D. Animal studies.

Studies in animal models have shown that CBL1 is capable of prolonging skin allograft survival in Rhesus monkeys without an adverse side effect. Monkeys were 5-6 Kg and received up to 0.45 ml (5mg) daily for 16 to 22 days of CBL1 ascites with a titer of $1:10^4$. The six monkeys treated with CBL1 had no side effects and had significant prolonged skin graft (size 4 x 4 cm on forearm) survival durations of 20 days over that of four untreated controls in which skin grafts survived 5 days. Differential blood counts taken every other day revealed no changes (Table 4) and all animals were healthy and well.

E. Human studies.

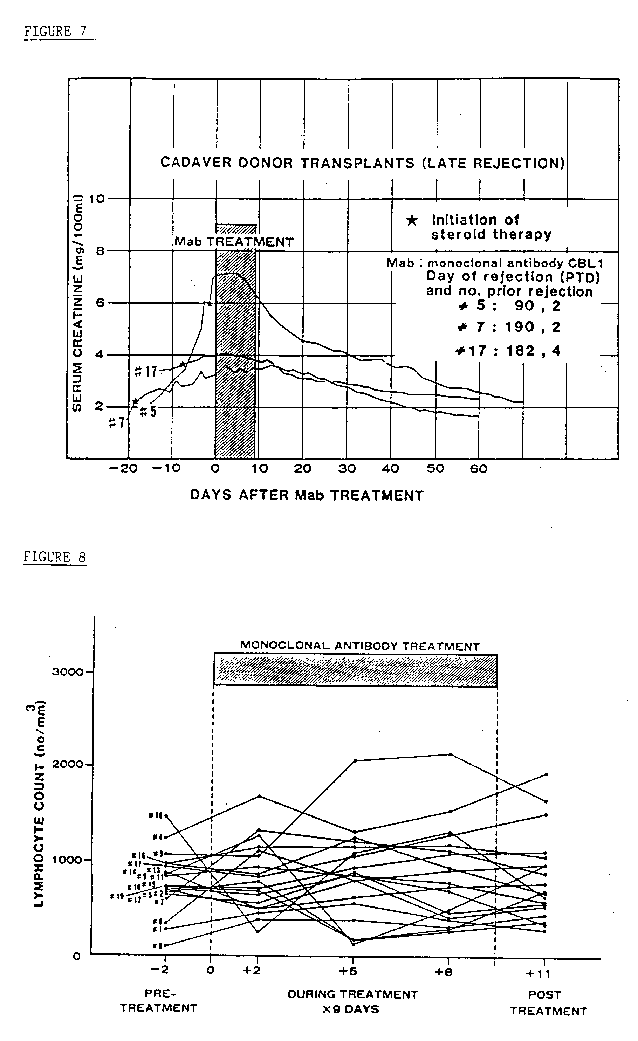

Nineteen patients with severe steroid resistant kidney graft rejections were treated with the monoclonal antibody. Five mg in 200 ml of saline was given intravenously on 9 successive days. None of the 19 patients developed fever, vomiting, treatment created infections or other side effects. All patients had previously been immunosuppressed with conventional antirejection steroid drugs which had lowered blood counts and failed to reverse transplant rejection. There was a dramatic reversal of graft rejection in 17 cases. The creatinine clearance which was dangerously high indicating kidney rejection (greater than 4mg/dL) showed a rapid decrease to normal levels starting on average 6 days following treatment, (Figs 5-7). With CBL1 treatment there was no fall in peripheral blood lymphocytes (fig 8), thrombocytopenia, and no reduction in peripheral blood monocyte count. Recurrence of graft rejection was uncommon after CBL1. One patient (#10, Fig 6) who had a recurrence of graft rejection 8 months after received a 2nd treatment with CBL1 that again reversed rejection. Apart from this case the majority of the treated patients have had functioning kidneys for more than 2 years. No other antibody or medication described in prior art has been able to reverse acute rejection without frequent reoccurrences of rejection.

A similar clinical study was done on three kidney transplant patients and six corneal graft patients at UCSD. Again conventional steroid rejection therapy had failed. Following i.v. CBL1 treatment with ascitic fluid dramatic reversal of graft rejection was seen in all cases without side effects. The dose of CBL1 ascites was 0.5 ml daily for 9 days in 100-200 ml of saline administered intravenously. The titer was $1:10^4$ to $1:10^5$. Blood counts and vital signs showed no adverse effects during or after treatment. In the case of the corneal graft rejection the patient's vision was very limited due to opaqueness caused by rejection. Following CBL1 therapy normal vision was restored in all cases.

EP 0 311 438 B1

Four patients with advanced metastatic cancer have been treated with CBL1, 5 mg per day for 10 days i.v. Tumor size was reduced following the treatment and no adverse side effects were noted. These studies are in their preliminary stages.

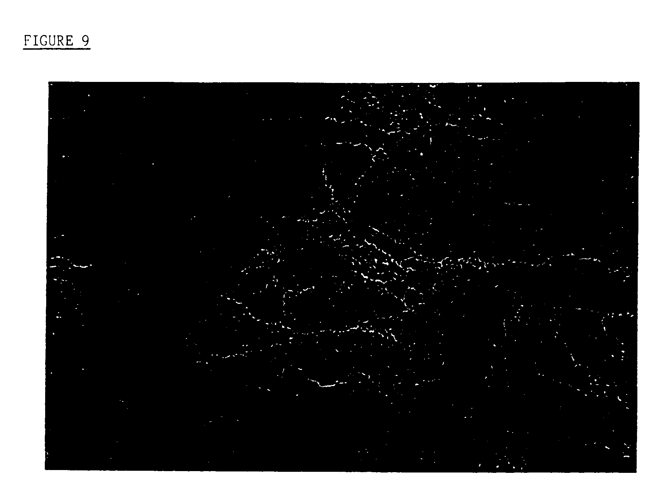

Case 1. A 66 year old male (EC) with metastatic colon cancer in the lymph nodes, spleen, abdomen and liver 3 years following surgery had developed an ulcer following extensive chemotherapy and was hospitalised for intravenous feeding. Following intravenously 0.5 ml CBL1 ascites daily for 10 days a large palpable abdominal tumor became necrotic and the skin lesion healed. Several weeks later he died of malnutrition while still on intravenous feeding. Tissue sections taken from autopsy tissue showed extensive necrosis of tumor cells but no visible damage to normal tissue. Fig 9 shows necrosis of malignant skin.

Case 2. A 48 year old male (GL) with a 2cm diameter squamous cell carcinoma in the nasopharyngeal area received six 0.5 cc doses of CBL1 ascites intramuscularly. One week following the treatment the tumor was no longer visible by endoscopy. Four weeks later a scan by magnetic resonance imaging revealed no evidence of the tumor in the nasopharynx. No evidence of side effects were observed.

Case 3. A male (JB) with colorectal cancer with metastasis to the lymph nodes and lower abdomen causing a large tumor abscess on the left groin and protruding rectal tumor was treated with CBL1 purified monoclonal antibody. Following ten daily treatments of 20 mg per day the tumor abscess healed completely and the protruding rectal tumor receded.

Case 4. A 58 year old male (JR) diagnosed as primary Dukes C colon cancer had lymph node and liver metastasis following surgical removal of the tumor in the colon. His CEA level following surgery was 16 ng / ml which indicated a poor prognosis. Following 5 months of chemotherapy his condition had not changed. 22 months following i.m. CBL1 treatment the lymph node metastasis recede and he is still alive and well with a single metastatic tumor in the liver.

9

TABLE 1

MONOCLONAL ANTIBODY REACTIONS AGAINST NORMAL AND MALIGNANT CELLS

| Normal cells - Type | # of samples | % positive |
|---|---|---|
| Peripheral blood T cells | 12 | < 2% |
| Peripheral blood B cells | 12 | < 2% |
| Peripheral blood granulocytes | 12 | < 2% |
| Peripheral blood platelets | 10 | < 2% |
| Peripheral blood monocytes | 15 | >80% |
| Peripheral blood activated lymphocytes | 6 | >60% |
| Malignant cells - Type | | |
| Acute myeloid leukemias | 25 | >95% |
| Acute lymphoid leukemias | 28 | >95% |
| Chronic myeloid blast crisis | 10 | >95% |
| Chronic lymphocytic leukemias | 20 | < 2% |
| Cultured leukemia cell lines, | | |
| Reh, CEM, HSB2, Daudi, Raji, | | |
| HL60, KG, JM, Molt 4, BJAB | 10 | >95% |
| Tumor cell lines | 16 | >95% |

() is number of different cell lines tested,

colorectal (3), lung (4), liver (1), breast (2),

prostate (1), kidney (1), esophagus (2), bladder (2).

| Tumor cells in frozen tissue sections | 20 | +ve by immunoperoxidase staining |
|---|---|---|

() is number of different carcinomas tested,

colon (6), rectal (1), esophageal (2), breast (5), kidney (1), meningioma (3), pancreatic (1), adrenal (1).

TABLE 2

| PERCENT POSITIVE CELLS IN CULTURE OF PHA STIMULATED VERSUS UNSTIMULATED PERIPHERAL BLOOD LYMPHOCYTES | | | | | |
|---|---|---|---|---|---|
| ANTIBODY | | PERCENTAGE CELLS WITH POSITIVE SURFACE STAINING | | PERCENTAGE CELLS WITH POSITIVE CYTOPLASMIC STAINING | |
| | | PHA | NO PHA | PHA | NO PHA |
| A. | CBL1 (a)* | 44 | 0 | 97 | 97 |
| | CBL1 (b) | 21 | 11 | 97 | 96 |
| | CBL1 (c) | 20 | 3 | 91 | 92 |
| B. | OKT9 (c) | 22 | 3 | 33 | <1 |
| | OKT9 (d) | n.d. | n.d. | 30 | 5 |

* Lower case letters identify the individual PBL donors.

TABLE 3

| RATES OF GROWTH OF TUMOR CELLS IN SERUM FREE CULTURE MEDIA IN THE PRESENCE OF GROWTH FACTOR AND MA | |
|---|---|
| ADDITIONS TO GROWTH MEDIUM | RATE OF GROWTH[1] |
| None | 0 |
| 20% Growth Medium (GM)[2] | + |
| 20% GM + CBL1[3] | 0 |
| 20% GM + B5[4] | + |
| Tumor cell lysate (TCL)[5] | + |
| TCL + CBL1 | 0 |
| TCL + B5 | + |
| Purified CAGF[6] | + |
| Purified CAGF + CBL1 | - |

1. 0 = no growth, + = cell density increases 2 fold each day (average)
2. Percentage of supernatant added from cells in log phase growth in serum free medium
3. 50ug purified MA
4. Control MA
5. $10 \times 10^6$ tumor cells lysed in 1 ml PBS by rapid freezing and thawing followed by centrifugation
6. Purified by ammonium sulfate precipitation and gel filtration

TABLE 4

| SKIN GRAFT SURVIVAL IN RHESUS MONKEYS TREATED WITH MONOCLONAL ANTIBODY CBL1 | | | | |
|---|---|---|---|---|
| EXP # | ANIMAL # | TITER | DOSE/LAMDA | SKIN GRAFT SURVIVAL |
| 1 | 17182 | $10^4$ | 300 | 16 days |
| 2 | 17083 | $10^4$ | 300 | 16 days |
| 3 | 17085 | $10^4$ | 300 | 15 days |
| 4 | 7633 | $10^4$ | 450 | 22 days |
| 5 | 7212 | $10^4$ | 450 | 20 days |
| 6 | 7253 | $10^4$ | 450 | 20 days |

**Claims**

1.  A partially purified antigen of vertebrate source that has the following properties:
    (a) it functions as an autocrine growth factor produced by tumor cells and activated lymphocytes,
    (b) it binds to the surface membrane of tumor cells and stimulates the growth of these cells and cells of the lymphoid series,
    (c) it is present on the cell membrane and within the cytoplasm of tumor cells and activated lymphocytes,
    (d) it is present in the cytoplasm of unstimulated normal peripheral blood lymphocytes but when these cells are stimulated by antigens or by mitogens, said antigen appears also on cell membrane,
    (e) it is present on lymphocytes activated in vitro by mitogens,
    (f) its molecular weight is approximately 15,000 daltons,
    (g) it is capable of binding to CBL1 monoclonal antibody which is produced by the hybridoma cell line having ATCC number HB 8214.

2.  An antigen according to claim 1 for use as a diagnostic or therapeutic agent.

3.  An antigen according to claim 1 for use as a growth factor to stimulate cell growth.

4.  An antigen according to claim 1 when coupled to a detectable label.

5.  An antigen according to claim 1 when coupled to an insoluble phase.

6.  An antigen according to claim 1 when coupled to a toxin or drug.

7.  An antigen according to claim 1 or 6 for use as an agent against diseases selected from:
    (a) solid tumors and acute leukemias,
    (b) autoimmune diseases,
    (c) transplant rejections,
    (d) allergic diseases.

8.  A method for assaying a biological specimen for the presence of an antigen defined in claim 1, or its cell receptor in tissues, on cells or in body fluids, the method comprising the steps of:
    incubating the specimen with labelled or unlabelled CBL1 antibody, and
    determining whether or not an immune complex is formed.

**Patentansprüche**

1.  Teilweise gereinigtes Antigen aus einer Vertebraten-Quelle, das die folgenden Eigenschaften aufweist:
    (a) es wirkt als autokriner Wachstumsfaktor, der von Tumorzellen und aktivierten Lymphocyten erzeugt wird,
    (b) es bindet sich an die Oberflächenmembran von Tumorzellen und stimuliert das Wachstum dieser Zellen und von Zellen der Lymphoid-Reihen,
    (c) es ist auf der Zellmembran und im Cytoplasma von Tumorzellen und aktivierten Lymphocyten vorhanden,
    (d) es ist im Cytoplasma von nichtstimulierten normalen Lymphocyten des peripheren Bluts vorhanden, wenn diese Zellen jedoch von Antigenen oder Mitogenen stimuliert werden, erscheint dieses Antigen auch auf der Zellmembran,
    (e) es ist auf Lymphocyten vorhanden, die in vitro von Mitogenen aktiviert wurden,
    (f) sein Molekulargewicht beträgt etwa 15 000 Dalton,
    (g) es kann sich an den monoklonalen Antikörper CBL1 binden, der von der Hybridomzellinie mit der ATCC-Nummer HB 8214 erzeugt wird.

2.  Antigen nach Anspruch 1 zur Verwendung als diagnostisches oder therapeutisches Mittel.

3.  Antigen nach Anspruch 1 zur Verwendung als Wachstumsfaktor für die Stimulation des Zellwachstums.

4.  Antigen nach Anspruch 1, wenn es an einen nachweisbaren Marker gekoppelt ist.

EP 0 311 438 B1

**5.** Antigen nach Anspruch 1, wenn es an eine unlösliche Phase gekoppelt ist.

**6.** Antigen nach Anspruch 1, wenn es an ein Toxin oder Medikament gekoppelt ist.

**7.** Antigen nach Anspruch 1 oder 6 zur Verwendung als Mittel gegen Krankheiten, die ausgewählt sind aus:
(a) feste Tumore und akute Leukämien
(b) Autoimmunkrankheiten
(c) Transplantatabstoßungen
(d) allergische Krankheiten.

**8.** Verfahren zur Untersuchung einer biologischen Probe nach dem Vorhandensein eines Antigens nach Anspruch 1 oder dessen Zellrezeptor in Geweben, auf Zellen oder in Körperflüssigkeiten, wobei dieses Verfahren die Schritte umfaßt:
Inkubieren der Probe mit einem markierten oder nichtmarkierten CBL1-Antikörper und
Bestimmen, ob ein Immunkomplex gebildet wurde oder nicht.

**Revendications**

**1.** Antigène partiellement purifié provenant d'un vertébré, qui présente les propriétés suivantes:
(a) il agit comme un facteur de croissance sécrété de façon autocrine, produit par des cellules tumorales et des lymphocytes activés,
(b) il se lie à la membrane de surface de cellules tumorales et stimule la croissance de ces cellules et de cellules de la série lymphoïde,
(c) il est présent sur la membrane cellulaire et à l'intérieur du cytoplasme de cellules tumorales et de lymphocytes activés,
(d) il est présent dans le cytoplasme de lymphocytes sanguins périphériques normaux non stimulés, mais lorsque ces cellules sont stimulées par des antigènes ou par des mitogènes, cet antigène apparaît aussi sur la membrane cellulaire,
(e) il est présent sur des lymphocytes activés in vitro par des mitogènes,
(f) son poids moléculaire est d'environ 15.000 daltons,
(g) il est capable de se lier à un anticorps monoclonal CBL1 qui est produit par la lignée cellulaire d'hybridome ayant le numéro ATCC HB 8214.

**2.** Antigène suivant la revendication 1, utilisable en tant qu'agent diagnostique ou thérapeutique.

**3.** Antigène suivant la revendication 1, utilisable en tant que facteur de croissance pour stimuler la croissance cellulaire.

**4.** Antigène suivant la revendication 1, lorsqu'il est couplé à un marqueur détectable.

**5.** Antigène suivant la revendication 1, lorsqu'il est couplé à une phase insoluble.

**6.** Antigène suivant la revendication 1, lorsqu'il est couplé à une toxine ou à un médicament.

**7.** Antigène suivant les revendications 1 ou 6, utilisable en tant qu'agent contre des maladies sélectionnées parmi:
(a) des tumeurs solides et des leucémies aiguës,
(b) des maladies auto-immunes,
(c) des rejets de transplant,
(d) des maladies allergiques.

**8.** Méthode d'essai d'un spécimen biologique pour déterminer la présence d'un antigène défini suivant la revendication 1, ou son récepteur cellulaire dans des tissus, sur des cellules ou dans des fluides corporels, comprenant les étapes de :
incubation du spécimen avec un anticorps CBL1 marqué ou non marqué, et
détermination de la formation ou non d'un complexe immun.

13

FIGURE 1

FIGURE 2

FIGURE 3

FIGURE 4

FIGURE 5

FIGURE 6

FIGURE 7

CADAVER DONOR TRANSPLANTS (LATE REJECTION)

DAYS AFTER Mab TREATMENT

FIGURE 8

MONOCLONAL ANTIBODY TREATMENT

EP 0 311 438 B1

FIGURE 9